(19) Europäisches Patentamt
European Patent Office
Office européen des brevets

(11) EP 1 504 759 A1

(12) **EUROPEAN PATENT APPLICATION**
published in accordance with Art. 158(3) EPC

(43) Date of publication:
**09.02.2005 Bulletin 2005/06**

(21) Application number: **03752896.5**

(22) Date of filing: **14.05.2003**

(51) Int Cl.$^7$: **A61K 31/496**, A61K 31/506, A61P 13/00, A61P 13/02, C07D 239/42, C07D 277/42

(86) International application number:
**PCT/JP2003/006003**

(87) International publication number:
**WO 2003/097060 (27.11.2003 Gazette 2003/48)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **15.05.2002 JP 2002140500**
**27.12.2002 JP 2002378797**

(71) Applicant: **Nippon Kayaku Kabushiki Kaisha**
**Tokyo 102-8172 (JP)**

(72) Inventors:
- **KOGA, Ichiro**
  **Saitama-shi, Saitama 331-0061 (JP)**
- **YAMAZAKI, Hiroko**
  **Kita-ku, Tokyo 114-0024 (JP)**
- **MATSUMOTO, Shin-ichi**
  **Saitama-shi, Saitama 338-0031 (JP)**
- **MATSUMOTO, Tetsuya**
  **Saitama-shi, Saitama 338-0001 (JP)**

(74) Representative:
**Gille Hrabal Struck Neidlein Prop Roos**
**Patentanwälte**
**Brucknerstrasse 20**
**40593 Düsseldorf (DE)**

(54) **ORAL THERAPEUTIC OR PREVENTIVE DRUGS FOR POLLAKIURIA AND URINARY INCONTINENCE OR ORAL SLEEP INDUCERS, CONTAINING TROPOLONE DERIVATIVES**

(57)

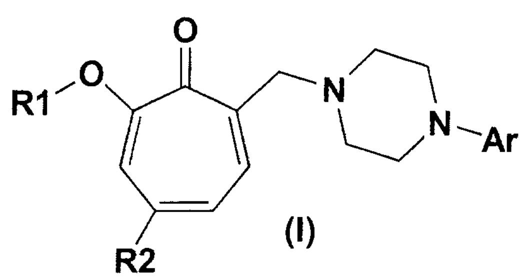

(I)

Oral therapeutic or preventive drugs for pollakiuria and urinary incontinence or oral sleep inducers, which contain as the active ingredient compounds represented by the general formula (I) or pharmacologically acceptable salts thereof and exhibit excellent peroral absorbability: (I) wherein R1 is hydrogen or linear or branched alkyl having 1 to 3 carbon atoms: R2 is hydrogen or isopropyl: and Ar is an un-substituted heteroaromatic group having two heteroatoms.

EP 1 504 759 A1

## Description

### BACKGROUND OF THE INVENTION

The present invention belongs to a technical field of therapeutic or preventive. drugs, and especially relates to the oral therapeutic or preventive drugs for pollakiuria and urinary incontinence or oral sleep inducers. These drugs contain, as the active ingredient compounds, tropolone derivatives which exhibit excellent peroral absorbability.

In our aging society, pollakiuria, urinary incontinence and dementia have become social problems.

Our society demands medical care that aims for maintaining the good QOL (quality of life) in old age. Especially, pollakiuria and urinary incontinence reduce the QOL for the aged by restricting activity and leading to a heavy burden for nurses and helpers for care.

Anticholinergic drugs, direct smooth muscle relaxants and the like are used for treating pollakiuria and urinary incontinence. The direct smooth muscle relaxants are less effective than the anticholinergic drugs but the anticholinergic drugs have side effects such as hydrodipsia and anuresis. Furthermore, anticholinergic drugs have insufficient efficacy for treating urinary incontinence in elderly patients because the increase in the atropine-resistant contraction is observed as an age-induced change in the smooth muscle of the human bladder.

It has been disclosed in International Publication No. WO 99/00366 that the tropolone derivatives can be used as therapeutic or preventive drugs for pollakiuria and urinary incontinence. However, drugs described in this publication, for example 2-benzyloxy-4-isopropyl-7-[4-phenylpiperazinomethyl]-2,4,6-cycloheptatrien-1-one was effective by intravenous injection, but no concrete disclosure was made whether it is effective as an oral therapeutic or preventive drug for pollakiuria and urinary incontinence.

Since oral administration of the drugs is preferable when QOL is considered, oral therapeutic or preventive drugs for pollakiuria and urinary incontinence with excellent peroral absorbability are desired in the clinical practice at the present time.

Therefore one of the objectives of the present invention is to provide oral therapeutic or preventive drugs with excellent peroral absorbability for pollakiuria and urinary incontinence.

On the other hand, sleep disorders are also large social problems in modern society. Sleep inducers such as barbiturates and benzodiazepines are used in clinical practice for treating sleep disorders such as insomnia. However, barbiturates are not used widely at the present time because they have disadvantages such as toxicity and the development of drug tolerance and drug dependence. Since benzodiazepines have a wider range of therapeutic efficacy and are safer than barbiturates, they have been used to treat many patients. However, their side effects include development of drug dependency, interaction with alcohol, dysmnesia and hypomyotonia. Therefore safer sleep inducing drugs are desired.

An object of the present invention is to provide oral sleep inducers with excellent peroral absorbability and with fewer side effects.

### DISCLOSURE OF THE INVENTION

The present inventors carried out intensive research to achieve the aforementioned objective. As a result we discovered that tropolone derivatives demonstrate unexpectedly high bioavailability, are absorbed from the alimentary canal, and are highly effective in treatment or prevention of pollakiuria and urinary incontinence as well as in sleep inducement.

The present invention relates to;

(1) General formula (I)

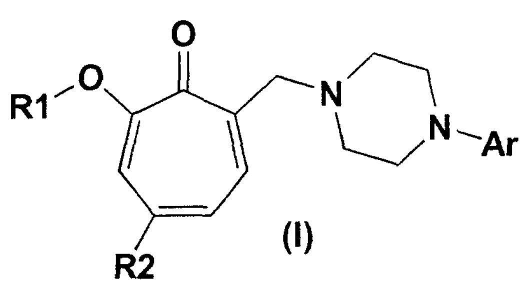

(I)

an oral therapeutic or preventive drugs for pollakiuria and urinary incontinence which contain as the active

ingredient compounds represented in the general formula (I) [wherein R1 represents a hydrogen atom or an alkyl group with 1 - 3 carbon atoms in a linear or branched carbon chain, R2 represents a hydrogen atom or an isopropyl group and Ar represents an un-substituted heteroaromatic group with 2 hetero atoms] or pharmacologically acceptable salts thereof;

(2) An oral therapeutic or preventive drug for pollakiuria and urinary incontinence according to (1), wherein Ar represents an un-substituted 2-pyrimidyl group or an un-substituted 2-thiazolyl group;

(3) An oral therapeutic or preventive drug for pollakiuria and urinary incontinence according to (1), wherein the compound expressed in general formula (I) is 4-isopropyl-2-methoxy-7-[4-(2-pyrimidyl)piperazinomethyl]-2,4,6-cycloheptatrien-1-one or 2-hydroxy-4-isopropyl-7-[4-(2-pyrimidyl)piperazinomethyl]-2,4,6-cycloheptatrien-1-one;

(4) An oral therapeutic or preventive drug for pollakiuria and urinary incontinence according to (1), wherein the pharmacologically acceptable salt is DL-tartrate or fumarate;

(5) An oral sleep inducer which contains the compound according to any one of (1) - (4) or pharmacologically acceptable salts thereof as an active ingredient compound;

(6) A serotonin 1 A antagonist which contains the compound according to any one of (1) - (4) or pharmacologically acceptable salts thereof as an active ingredient compound;

(7) 4-isopropyl-2-methoxy-7-[4-(2-pyrimidyl)piperazinomethyl]-2,4,6-cycloheptatrien-1-one or 2-hydroxy-4-isopropyl-7-[4-(2-pyrimidyl)piperazinomethyl]-2,4,6-cycloheptatrien-1-one or 2-hydroxy-4-isopropyl-7-[4-(2-thiazolyl) piperazinomethyl]-2,4,6-cycloheptatrien-1-one or pharmacologically acceptable salts thereof;

(8) 4-isopropyl-2-methoxy-7-[4-(2-pyrimidyl)piperazinomethyl]-2,4,6-cycloheptatrien-1-one 2 DL-tartrate or 4-isopropyl-2-methoxy-7-[4-(2-pyrimidyl)piperazinomethyl]-2,4,6-cycloheptatrien-1-one 1 fumarate.

## BEST MODE FOR CARRYING OUT THE INVENTION

Because the compounds expressed in the general formula (I) described above or pharmacologically acceptable salts thereof used for the present invention showed an affinity to the serotonin 1A (one of the intracerebral amines) receptor and possessed anti-serotonin 1A antagonistic activity, it is expected that these compounds are effective as therapeutic or preventive drugs for pollakiuria and urinary incontinence or sleep inducers and the like.

The oral therapeutic or preventive drugs for pollakiuria and urinary incontinence or the oral sleep inducers in the present invention contain, as active ingredient compounds, represented in the general formula (I) described above or pharmacologically acceptable salts thereof. In the compounds represented in the general formula (I) used in the present invention, R1 represents an hydrogen atom or an alkyl group with 1 - 3 carbon atoms in a linear or branched carbon chain, R2 represents a hydrogen atom or an isopropyl group and Ar represents an un-substituted heteroaromatic group with 2 hetero atoms.

The alkyl group with 1 - 3 carbon atoms in a linear or branched chain can be concretely, for example, a methyl group, an ethyl group, a n-propyl group or an isopropyl group. R2 is preferably an isopropyl group.

The 2 hetero atoms in the un-substituted heteroaromatic group in Ar can be two of the same or different atoms selected from a group consisting of nitrogen, oxygen and sulfur atoms. The preferred heteroaromatic groups can be, for example, 3-pyridazinyl group, 2-pyrimidyl group, 2-pyrazinyl group, 2-thiazolyl group, 2-oxazolyl group or 2-imidazolyl group. Especially preferable are 2-pyrimidyl group or 2-thiazolyl group.

The compounds expressed in the general formula (I) are exemplified in Table 1. In Table 1, H represents a hydrogen atom, Me represents a methyl group, Et represents an ethyl group, n-Pr represents a n-propyl group and i-Pr represents an isopropyl group.

Table 1

| Compound | R1 | R2 | - Ar |
|---|---|---|---|
| (1) | H | H | 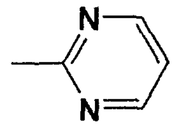 (2-pyrimidyl) |
| (2) | Me | H | 2-pyrimidyl |
| (3) | H | i-Pr | 2-pyrimidyl |
| (4) | Me | i-Pr | 2-pyrimidyl |
| (5) | Et | i-Pr | 2-pyrimidyl |
| (6) | n-Pr | i-Pr | 2-pyrimidyl |
| (7) | i-Pr | i-Pr | 2-pyrimidyl |
| (8) | H | i-Pr | 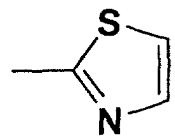 (2-thiazolyl) |
| (9) | Me | i-Pr | 2-thiazolyl |

Among the compounds represented by the general formula (I), a preferred compound is 4-isopropyl-2-methoxy-7-[4-(2-pyrimidyl)piperazinomethyl]-2,4,6-cycloheptatriencycloheptatrien-1-one (Compound (4)) or 2-hydroxy-4-isopropyl-7-[4-(2-pyrimidyl)piperazinomethyl]-2,4,6-cycloheptatrien-1-one (Compound (3)).

The compounds represented in the general formula (I) can be synthesized by the production method described in International Publication No. WO99/00366 but the present invention is not restricted to this method.

For example, the production routes are shown below for 4-isopropyl-2-methoxy-7-[4-(2-pyrimidyl)piperazinomethyl]-2,4,6-cycloheptatrien-1-one, and 2-hydroxy-4-isopropyl-7-[4-(2-pyrimidyl)piperazinomethyl]-2,4,6-cycloheptatrien-1-one.

Production routes

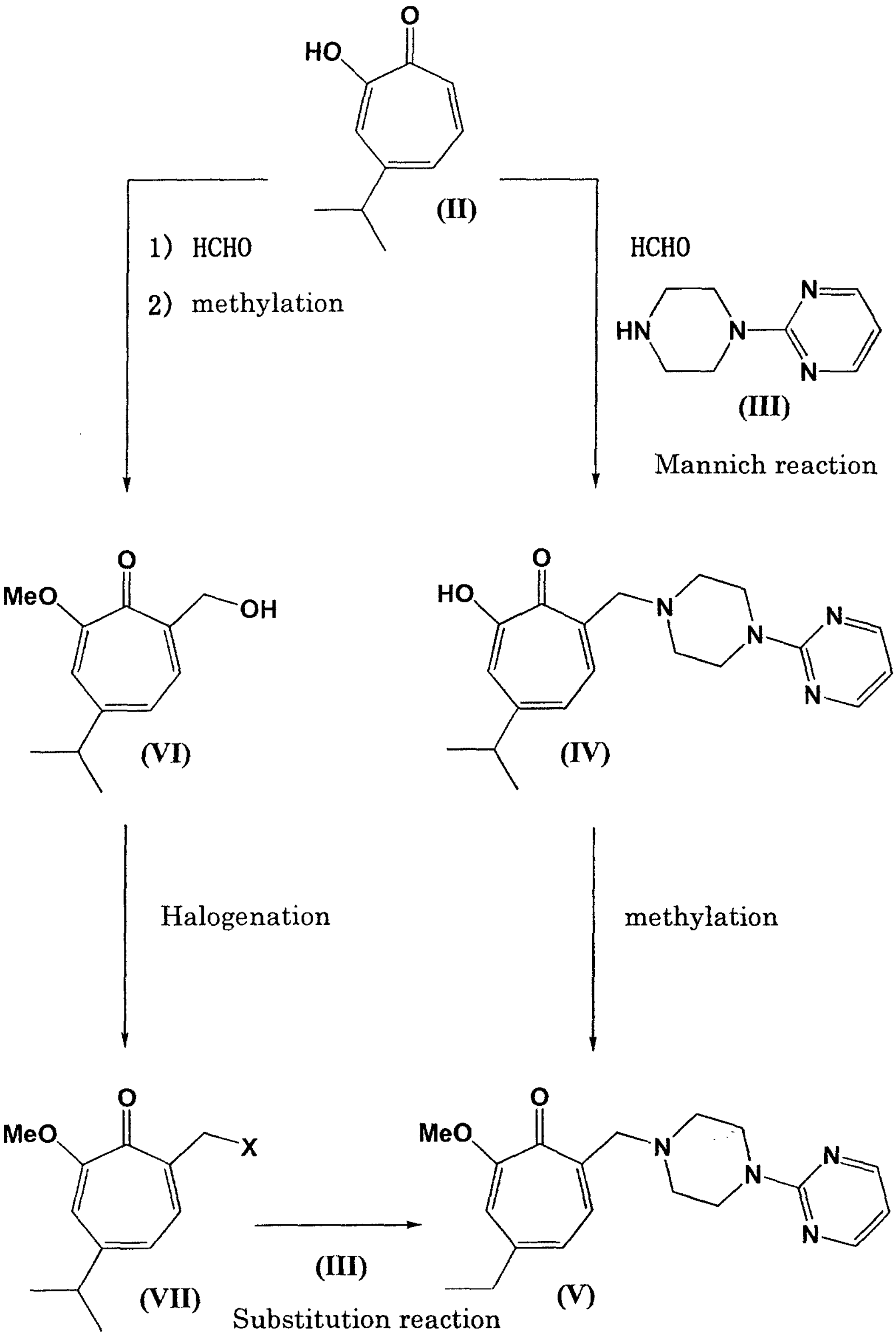

2-Hydroxy-4-isopropyl-7-[4-(2-pyrimidyl)piperazinomethyl]-2,4,6-cycloheptatrien-1-one (IV) is produced by the Mannich reaction of commercially available hinokitiol (2-hydroxy-4-isopropyl-2,4,6-cycloheptatorien-1-one)(II) with pyrimidylpiperazine (III).

The amount of pyrimidylpiperazine used in the reaction is 0.5 - 10 equivalence of hinokitiol, preferably 0.8 - 2.0 equivalence, more preferably 0.8 - 1.2 equivalence.

Aqueous formaldehyde is used at 0.5 - 30 equivalence, preferably 0.8 - 2 equivalence.

The reaction solvents are not specified but alcohols (methanol, ethanol, propanol and etc.), esters (methyl acetate, ethyl acetate and etc.), ethers (ethyl ether, isopropyl ether, tetrahydrofuran, dioxane and etc.), aromatic hydrocarbons (benzene, toluene, xylene and etc.), aliphatic hydrocarbons (pentane, hexane and etc.), water and acetic acid can be used. It is preferable to use alcohols.

As acids, mineral acids such as hydrochloric acid, sulfuric acid and the like and organic acids such as p-toluenesulfonic acid, acetic acid and the like can be used but it is preferable to use acetic acid.

The amount of acid used in the reaction is in the range of 0.01 equivalence to the amount of solvent, and is preferably 1 - 2 equivalence.

The reaction temperature can be in the range of-10 deg C to around the boiling point of the solvent, preferably in the range of room temperature to around the boiling point of the solvent. The reaction time can be for 0.5 - 24 hours, preferably for 2 - 8 hours.

After the reaction, the target compounds can be obtained by the general purification methods; cooling the reaction mixture to crystallize and collecting the crystals by filtration, or concentrating and recrystallizing, or purificating by column chromatography and the like.

Methylation of hydroxyl groups can be carried out by adding a base and a methylating agent.

Lithium carbonate, sodium carbonate, potassium carbonate, cesium carbonate, sodium hydroxide, potassium hydroxide, lithium hydroxide, triethylamine, pyridine and the like can be used as the base and the amount used can be 0.5 - 10 equivalence, preferably 0.8 - 2 equivalence.

Methyl iodide, methyl bromide, dimethyl sulfate and the like can be used as the methylating agent and the amount used is 0.5 - 10 equivalence, preferably 0.8 - 3 equivalence.

The reaction solvents are not specified but ketones (acetone, methyl ethyl ketone, methyl isobutyl ketone and etc.), esters (methyl acetate, ethyl acetate and etc.), ethers (ethyl ether, isopropyl ether, tetrahydrofuran, dioxane and etc.), aliphatic hydrocarbons (pentane, hexane and etc.), halogenated solvent (methylene chloride, chloroform, 1,2-dichloroethane and etc.), dimethylformamide, dimethylsulfoxide and the like can be used.

It is preferable that the reaction temperature can be in the range of-10 deg C to around the boiling point of the solvent, and the reaction time can be for 0.5 - 48 hours.

After the reaction, general purification method such as recrystallization or column chromatography can be used and by this procedure 4-isopropyl-2-methoxy-7-[4-(2-pyrimidyl)piperazinomethyl]-2,4,6-cycloheptatrien -1-one (V) is produced.

Also the compound can be synthesized according to the method described in Japanese translation of PCT application Publication No. 6-509318.

That is, the compound (VI) is obtained by reacting hinokitiol with formaldehyde and then carrying out methylation. Further, the compound (VI) is converted to the compound (VII) by halogenation and then the final product is produced by the substitution reaction with pyrimidylpiperazine.

The pharmacologically accepted salts of the compounds used in the present invention are salts with mineral acids such as hydrochloric acid, sulfuric acid and the like, with organic sulfonic acids such as methanesulfonic acid, p-toluenesulfonic acid and the like, with organic carboxylic acids such as acetic acid, propionic acid, succinic acid, lactic acid, tartaric acid, malic acid, fumaric acid, maleic acid and the like, with alkali metals such as sodium, potassium and the like, and with alkali earth metals such as calcium, magnesium and the like.

The salts with DL-tartaric acid or fumaric acid are not hygroscopic, and more concretely, 4-isopropyl-2-methoxy-7-[4-(2-pyrimidyl)piperazinomethyl]-2,4,6-cycloheptatrien-1-one 2DL-tartrate or 4-isopropyl-2-methoxy-7-[4-(2-pyrimidyl)piperazinomethyl]-2,4,6-cycloheptatrien-1-one 1 fumarate are preferable as the drug product because they do not cause a problem in guaranteeing the content of the active ingredient.

In the present invention, an oral drug means a drug with peroral absorbability, wherein peroral absorbability means that the drug shows pharmacological activities when administered orally with normal dose and usage without showing side effects.

Peroral absorbability involves absorption from the alimentary canal, transfer to the blood stream, the first-pass effect and the distribution to the target organs.

In the experiments described later, the oral therapeutic or preventive drugs in the present invention for pollakiuria and urinary incontinence or oral sleep inducers administered in the duodenum of rats show activity on rhythmic bladder contractions, and bioavailability by rats was demonstrated. The oral administration of these drugs induced sleep in dogs. These data suggest that the drugs of the present invention have an excellent peroral absorbability and

pharmacological activity as an oral drug.

The oral therapeutic or preventive drugs in the present invention for pollakiuria and urinary incontinence or sleep inducers can be used singly or as components with other drugs or medical additives.

For example, the formulation of oral drugs can be made in the forms of powder, granule, tablet or capsule by combining with pharmacologically acceptable carriers, excipients and diluents. The contents in the formulation for the compounds of the present invention or pharmacologically acceptable salts thereof can be 0.01 - 100 percent by weight, preferably 0.1 - 90 percent by weight and the rest is the range of the medical additives. The most preferred route for administration is oral (including buccal and sublingual). Dosage can be varied according to the age of the patient and the symptoms to be treated; for example, the oral dosage for adults can be 0.1 mg - 2000 mg per day, preferably 0.5 mg - 100 mg per day, which can be divided and administered several times a day.

In the present invention, a preventive drug means the formulation given to elderly people who has a high incidence of pollakiuria and urinary incontinence before the symptoms develop. The medicinal formulations administered based on slight symptoms or suitable indexes before the onset of the symptoms are also included.

The present invention includes
4-isopropyl-2-methoxy-7-[4-(2-pyrimidyl)piperazinomethyl]-2,4,6-cycloheptatrien-1-one (the compound (4) in Table 1) wherein R1 is a methyl group, R2 is an isopropyl group and Ar is a 2-pyrimidyl group in the general formula (I), 2-hydroxy-4-isapropyl-7-[4-(2-pyrimidyl)piperazinomethyl]-2,4,6-cycloheptatrien-1-one (the compound (3) in Table 1) wherein R1 is a hydrogen atom, R2 is an isopropyl group and Ar is a 2-pyrimidyl group,
2-hydroxy-4-isopropyl-7-[4-(2-thiazolyl)piperazinomethyl]-2,4,6-cycloheptatrien-1-one (the compound (8) in Table 1) wherein R1 is a hydrogen atom, R2 is an isopropyl group and Ar is a 2-thiazolyl group, or pharmacologically acceptable salts thereof.

Next, the results of the biological evaluation for the present invention are shown as the test example 1, the test example 2, the test example 3 and the test example 4.

Test drug 1: 4-isopropyl-2-methoxy-7-[4-(2-pyrimidyl)piperazinomethyl]-2,4,6-cycloheptatrien-1-one 1 hydrochloride (the compound for Example 4: hydrochloric acid salt of the compound (4) in Table 1).

Test drug 2: 2-hydroxy-4-isopropyl-7-[4-(2-pyrimidyl) piperazinomethyl]-2,4,6-cycloheptatrien-1-one 1 hydrochloride (the compound for Example 2: hydrochloric acid salt of the compound (3) in Table 1).
Control drug: 2-benzyloxy-4-isopropyl-7-[4-phenylpiperazinomethyl]-2,4,6-cycloheptatrien-1-one 1 fumarate.

The compound produced by the method described in International Pablication No. WO99/00366 was used as the control drug.

Test Example 1. Effect on the rhythmic bladder contraction of rats

The inhibitory effect on the micturition reflex was evaluated by the effect on the rhythmic bladder contraction according to the method of Kaseda et al. (Rinshyoseiri, 5, 540 - 547 (1975)).

METHOD

SD-IGS strain male rats were anesthesized by intraperitonial administration of urethane (1g/kg). After exposing the bladder of the animal through a incision of the abdomen, a small incision was made on the top of the bladder from where a rubber balloon was inserted.

The balloon was attached to a polyethylene tube connecting to a three way stopcock. The rest two ways of the stopcock were connected to a syringe for infusing water to the balloon and a transducer to measure the internal pressure of the bladder.

The test animal was left for a while and then water was injfused into the balloon until rhythmic contraction started. The change in the internal pressure of the bladder was recorded continuously by a polygraph. Each test drug was dissolved in de-ionized water and administered through a catheter inserted in the duodenum.

The effect of the drug was expressed as the inhibition ratio of the frequency of the rhythmic contraction of the bladder obtained by following formula

Inhibition rate (%) = 100 - {the frequency of the rhythmic contraction during 120 minutes following the administration (converted to the frequency per unit time) / the frequency of the rhythmic contraction 20 minutes immediately before the administration (converted to the frequency per unit time) x 100}

.

Table 2

| Inhibition rate of the rhythmic contraction frequency of the bladder (%) (the number in the parentheses shows the number of animals) | | | |
|---|---|---|---|
| Drug | Dose (mg/kg) 12.5 | 6.25 | 3.13 |
| Test drug 1 | N. T. | 48.89+/-5.43*(n=4) | 43.48+/-4.98*(n=3) |
| Test drug 2 | 59.08+/-3.18*(n=3) | 26.84+/-5.33 (n=4) | N. T. |
| | | | |
| Control drug | 4.81+/-4.09 (n=6) | N. T. | N. T. |
| [de-ionized water (Negative Control); 0.02 +/- 3.80 (n=5)]<br>N.T.: Not Tested<br>*: P<0.05 Student t test vs de-ionized water data | | | |

RESULT

The test drugs 1 and 2 showed a marked inhibitory activity on the frequency of the rhythmic bladder contraction by intra-duodenal administration of 6.25 mg/kg but the control drug did not show significant activity even at the dose of 12.5 mg/kg. Since orally administered drugs are mainly absorbed at the alimentary canal and are mainly metabolized at the liver, it was demonstrated that the compounds of the present invention provide effective pharmacological activity via oral administration.

Test Example 2. Measurement of bioavailability by oral administration in rats.

The intravenous administration group and the oral administration group consisted of 3 rats each (6 week-old male SD-IGS strain (Japan Charles River Co.)).

METHOD

Each test drug was dissolved in physiological saline or ultra-pure water and administered to the rats intravenously or orally at 6.25 mg/kg. The blood samples were collected periodically and the plasma were prepared by the routine procedures. After deproteinization, the concentration of each test drug was measured by the LC/ESI(+) SRM method (liquid chromatography/electrospray-ionization (+) selected reaction monitoring method) and the bioavailability was calculated as the ratio (percent) of AUC (area under the blood concentration-time curve) of the oral administration and AUC of the intra-venous administration.

Table 3

| Bioavailability | |
|---|---|
| Drug | Bioavailability (%) |
| Test drug 1 | 57% |
| Test drug 2 | 31% |
| | |
| Control drug | 6% |

RESULT

The bioavailability of the test drugs 1 and 2 was about 30 - 60%, suggesting that they can be used for oral administration, but the bioavailability of the control drug was 6% indicating it is unsuitable for oral administration.

Test Example 3. Observation of sleep-inducing symptoms in dogs after the oral administration of the drugs.

METHODS

Male dogs (Beagles) were orally administered with capsules filled with the test drug 1 and the symptoms were observed. The dosages were 3 mg/kg, 10 mg/kg, 30 mg/kg and 100 mg/kg.

RESULTS

After the oral administration of 3 mg/kg, no abnormal symptom was recognized during the observation period (n=2). In the group of more than 10 mg/kg, drowsiness was observed (somewhat falling asleep, the second state from the most awake state when levels of consciousness are divided into 4 states) after 20 - 30 minutes of the administration. During the drowsiness state, arousal response was observed in response to touch or sound stimuli, but after arousal reactions the animal became to the state of drowsiness.

The drowsiness was observed for 142 and 318 minutes for doses of 30 mg/kg and 100 mg/kg, respectively, which tended to be dose-dependent with the administered dose. Since the drug concentration in the plasma increased dose-dependently, it would appear that the test drug 1 was absorbed and caused the drowsiness pharmacologically. No abnormal behavior other than drowsiness was observed.

Test Example 4. Serotonin 1A antagonism activity.

METHODS

The affinity of the test drug 1 to the human serotonin 1 A receptor was measured according to the method by Mulheron et al. (J. G. Mulheron et al., J. Biol. Chem., 269, 12954-12962 (1994)) by using the CHO cells transfected with the human serotonin 1A receptor gene. The concentration of the test drug was set at $10^{-6}$ M which was almost the same as the plasma concentration in the rats administered orally with 6.25 mg/kg of the test drug 1 at which the rhythmic bladder contraction was inhibited significantly.

RESULTS

The $10^{-6}$ M test drug 1 competitively inhibited the binding of 0.3 nM of 8-OH-DPAT to the human serotonin 1A receptor by 77%, suggesting that the drug 1 has a strong affinity to the human serotonin 1A receptor.

Next, the results of the evaluation of the hygroscopic characteristics of the drugs in the present invention are shown.

Test Example 5. Hygroscopic evaluation

Test sample 1 (the compound in the Example 5): 4-isopropyl-2-methoxy-7-[4-(2-pyrimidyl)piperazinomethyl]-2,4,6-cycloheptatrien-1-one 2 DL-tartrate

Test sample 2 (the compound in the Example 6): 4-isopropyl-2-methoxy-7-[4-(2-pyrimidyl) piperazinomethyl]-2,4,6-cycloheptatrien-1-one 1 fumarate

METHODS

Fifty mg of the each test sample was weighed exactly and placed in a closed container in which the relative humidity was kept at 75% and the bottom chamber was filled with saturated brine. The change in the weight of the test samples were measured periodically and the results are shown in Table 4.

Table 4.

| Weight change (mg) in 75% relative humidity. | | |
|---|---|---|
| | Test sample 1 | Test sample 2 |
| Storage time (hours) | | |
| 0 | 0.0 | 0.0 |

Table 4. (continued)

| Weight change (mg) in 75% relative humidity. | | |
|---|---|---|
| | Test sample 1 | Test sample 2 |
| Storage time (hours) | | |
| 2 | 0.2 | 0.3 |
| 4 | 0.2 | 0.4 |
| 6 | 0.1 | 0.1 |
| 103 | 0.1 | 0.1 |
| 170 | 0.1 | 0.2 |
| | | |
| 26 days | 0.1 | 0.2 |

RESULTS

No substantial increase was observed in the weight of the test sample 1 or 2 at 75% relative humidity for 26 days confirming that these salts have extreme low hygroscopicity.

**Examples**

The present invention is explained concretely with examples but the present invention is not limited by these examples. The NMR values in the examples were values (ppm) obtained by using tetramethylsilane as internal standard.

Example 1. Synthesis of 2-hydroxy-4-isopropyl-7-[4-(2-pyrimidyl)piperazinomethyl]-2,4,6-cycloheptatrien-1-one (compound (3) in Table 1)

Hinokitiol (3.3 g, 20 mmol), 1-(2-pyrimidyl)piperazine (3.3 g, 20 mmol) and acetic acid (1.1 mL, 20 mmol) were mixed with 10 mL of methanol and then added with 37% aqueous formalin (1.6 mL, 20 mmol). After stirring at room temperature for about 15 hours, 2-hydroxy-4-isopropyl-7-[4-(2-pyrimidyl)piperazinomethyl]-2,4,6-cycloheptatrien-1-one (3.6 g, 10.5 mmol, 53%) was obtained by collecting the precipitated crystals by filtration.
MS (m/z): 341$[M+H]^+$
$^1$H-NMR (200 MHzFT, TMS, $CDCl_3$)
1.29 (6H, d, J=6.9 Hz)
2.61 (4H, t, J=5.1 Hz)
2.92 (1H, qui, J=6.9 Hz)
3.75 (2H, s)
3.87 (4H, t, J=5.1 Hz)
6.49 (1H, t, J=4.8 Hz)
7.03 (1H, dd, J=10.6, 1.7 Hz)
7.35 (1H, d, J=1.7 Hz)
7.85 (1H, d, J=10.6 Hz)
8.31 (2H, d, J=4.8 Hz)

Example 2. Synthesis of 2-hydroxy-4-isopropyl-7-[4-(2-pyrimidyl)piperazinomethyl]-2,4,6-cycloheptatrien-1-one 1 hydrochloride

2-Hydroxy-4-isopropyl-7-[4-(2-pyrimidyl)piperazinomethyl]-2,4,6-cycloheptatrien-1-one (7.6 g, 22 mmol) obtained in Example 1 was dissolved in 65 mL ethyl acetate and then 4 N HCl-ethyl acetate solution (8.3 mL, 33 mmol) was added dropwise. After stirring at room temperature for 1 hour, crude crystals were collected by filtration. The crude crystals were suspended in 50 mL acetone, stirred at room temperature for 2 hours and then 2-hydroxy-4-isopropyl-7-[4-(2-pyrimidyl)piperazinomethyl]-2,4,6-cycloheptatrien-1-one 1 hydrochloride (8.4 mg) was obtained by filtration.
$^1$H-NMR (200 MHzFT, TMS, $CD_3OD$)
1.31 (6H, d, J=6.8 Hz)

3.01 (1H, qui, J=6.8 Hz)
3.2 - 3.8 (8H)
4.50 (2H, s)
6.82 - 6.89 (1H, m)
7.15 (1H, dd, J=10.0, 1.6 Hz)
7.46 (1H, d, J=1.6 Hz)
7.85 (1H, d, J=10.0 Hz)
8.48 - 8.52 (2H, m)

Example 3. Synthesis of 4-isopropyl-2-methoxy-7-[4-(2-pyrimidyl)piperazinomethyl]-2,4,6-cycloheptatrien-1-one (compound (4) in Table 1)

2-Hydroxy-4-isopropyl-7-[4-(2-pyrimidyl)piperazinomethyl]-2,4,6-cycloheptatrien-1-one (3.6 g, 10.5 mmol) obtained in Example 1 was dissolved in 20 mL dimethylformamide. Cesium carbonate (4.8 g, 13.6 mmol) and methyl iodide (1.8 g, 12.6 mmol) were added and followed by stirring at room temperature for about 15 hours. Ethyl acetate was added, and the reaction mixture was washed with water, dried with magnesium sulfate and concentrated under reduced pressure after filtration. 4-Isopropyl-2-methoxy-7-[4-(2-pyrimidyl)piperazinomethyl]-2,4,6-cycloheptatrien-1-one (0.20 g, 0.57 mmol, 5%) was obtained by purifying the residue by silica gel column chromatography (eluent; hexane:ethylacetate = 1:2).
MS (m/z): 355$[M+H]^+$
$^1$H-NMR (200 MHzFT, TMS, $CDCl_3$)
1.30 (6H, d, J=6.8 Hz)
2.60 (4H, t, J=5.1 Hz)
2.89 (1H, qui, J=6.8 Hz)
3.69 (2H, s)
3.87 (4H, t, J=5.1 Hz)
3.95 (3H, s)
6.48 (1H, t, J=4.7 Hz)
6.72 (1H, br)
6.84 - 6.92 (1H, m)
7.79 (1H, d, J=9.5 Hz)
8.31 (2H, d, J=4.7 Hz)

Example 4. Synthesis of 4-isopropyl-2-methoxy-7-[4-(2-pyrimidyl)piperazinomethyl]-2,4,6-cycloheptatrien-1-one 1 hydrochloride

4-Isopropyl-2-methoxy-7-[4-(2-pyrimidyl)piperazinomethyl]-2,4,6-cycloheptatrien-1-one (1.7 g, 4.8 mmol), obtained in Example 3 was mixed with 40 mL ethyl acetate and then 4N hydrochloric acid-dioxane solution (1.45 mL, 5.8 mmol) was added dropwise. The crude crystals were collected by filtration, mixed with 16 mL acetone, stirred at room temperature for 3 hours and then 4-isopropyl-2-methoxy-7-[4-(2-pyrimidyl)piperazinomethyl]-2,4,6-cycloheptatrien-1-one 1 hydrochloride (1.6 g) was obtained by filtering the crystals.
$^1$H-NMR (200 MHzFT, TMS, $CD_3OD$)
1.34 (6H, d, J=6.8 Hz)
3.31 (1H, qui, J=6.8 Hz)
3.1 - 3.7 (8H)
4.04 (3H, s)
4.41 (2H, s)
6.73 (1H, t, J=4.8 Hz)
7.1 - 7.2 (2H)
7.85 (1H, d, J=9.3 Hz)
8.41 (2H, d, J=4.8 Hz)

Example 5. Synthesis of 4-isopropyl-2-methoxy-7-[4-(2-pyrimidyl)piperazinomethyl] -2,4,6-cycloheptatrien-1-one 2 DL-tartrate

4-Isopropyl-2-methoxy-7-[4-(2-pyrimidyl)piperazinomethyl]-2,4,6-cycloheptatrien-1-one (1.0 g, 2.8 mmol), obtained in Example 3 was dissolved in 8 mL methanol, and 7 mL methanol solution of DL-tartaric acid (0.85 g, 5.6 mmol) was added dropwise. 4-Isopropyl-2-methoxy-7-[4-(2-pyrimidyl)piperazinomethyl]-2,4,6-cycloheptatrien-1-one 2

DL-tartrate (1.7 g, 2.6 mmol, 91%)was obtained by collecting the formed crystals by filtration.
$^1$H-NMR (200 MHzFT, TMS, $CD_3OD$)
1.33 (6H, d, J=6.8 Hz)
3.04 (1H, qui, J=6.8 Hz)
3.24 (4H, t, J=5.2 Hz)
4.0 - 4.1 (7H)
4.26 (2H, s)
4.48 (4H, s)
6.70 (1H, t, J=4.8 Hz)
7.0-7.1 (2H)
7.85 (1H, d, J=9.7 Hz)
8.38 (2H, d, J=4.8 Hz)

Example 6. Synthesis of 4-isopropyl-2-methoxy-7-[4-(2-pyrimidyl)piperazinomethyl]-2,4,6-cycloheptatrien-1-one 1 fumarate

4-Isopropyl-2-methoxy-7-[4-(2-pyrimidyl)piperazinomethyl]-2,4,6-cycloheptatrien-1-one (1.0 g, 2.8 mmol), obtained in Example 3 was dissolved in 15 mL ethanol, and 10 mL ethanol solution of fumaric acid (0.33g, 2.8 mmol) was added. After concentrating under reduced pressure, 1.5 mL water and 3 mL acetone were added, heated and dissolved. To this solution 10 mL acetone was added dropwise, the mixture was cooled to room temperature and 4-isopropyl-2-methoxy-7-[4-(2-pyrimidyl)piperazinomethyl]-2,4,6-cycloheptatrien-1-one 1 fumarate (0.80 g, 1.7 mmol, 60 %) was obtained by collecting the formed crystals by filtration.
$^1$H-NMR (200 MHzFT, TMS, $CD_3OD$)
1.33 (6H, d, J=6.8 Hz)
2.9 - 3.1 (5H)
3.9 - 4.0 (7H)
4.10 (2H, s)
6.67 (1H, t, J=4.8 Hz)
6.70 (2H, s)
7.0 - 7.1 (2H)
7.83 (1H, d, J=9.8 Hz)
8.37 (2H, d, J=4.8 Hz)

Example 7. Synthesis of 2-hydroxy-7-[4-(2-pyrimidyl)piperazinomethyl]-2,4,6-cycloheptatrien-1-one (Compound (1) in Table 1)

Commercially available tropolone (2.4 g, 20 mmol), 1-(2-pyrimidyl) piperazine (3.3 g, 20 mmol) and acetic acid (1.1 mL, 20 mmol) were mixed with 10 mL methanol and then 37% aqueous formalin (1.6 mL, 20 mmol) was added. After stirring at room temperature for 3 days, the reaction mixture was concentrated under reduced pressure and the residue was purified by silica gel column chromatography (eluent; methylene chloride:methanol=25:1). After suspending the purified fraction in methanol, 2-hydroxy-7-[4-(2-pyrimidyl)piperazinomethyl]-2,4,6-cycloheptatrien-1-one (1.1 g, 3.6 mmol, 18%) was obtained by filtration.
MS(m/z): 299$[M+H]^+$
$^1$H-NMR(200 MHzFT, TMS, $CDCl_3$)
2.62 (4H, t, J=5.1 Hz)
3.79 (2H, s)
3.88 (4H, t, J=5.1 Hz)
6.50 (1H, t, J=4.8 Hz)
7.0 - 7.5 (4H)
7.99 (1H, d, J=10.1 Hz)
8.32 (1H, d, J=4.8 Hz)

Example 8. Synthesis of 2-hydroxy-4-isopropyl-7-[4-(2-thiazolyl)piperazinomethyl]-2,4,6-cycloheptatrien-1-one (Compound (8) in Table 1)

Hinokitiol (0.29 g, 1.8 mmol), 1-(2-thiazolyl) piperazine (0.30 g, 1.8 mmol) and acetic acid (0.1 mL, 1.8 mmol) were mixed with 10 mL of methanol and then 37% aqueous formalin (0.16 mL, 18 mmol) was added. After stirring at room temperature overnight, 2-hydroxy-4-isopropyl-7-[4-(2-thiazolyl)piperazinomethyl]-2,4,6-cycloheptatrien-1-one

(0.3 g, 0.87 mmol, 49%) was obtained by collecting the formed crystals by filtration.
MS (m/z):346[M+H]$^+$
$^1$H-NMR (200 MHzFT, TMS, $CD_3OD$)
1.30 (6H, d, J=6.9 Hz)
3.01 (1H, qui, J=6.9 Hz)
3.3 - 3.9 (8H)
4.49 (2H, s)
6.92 (1H, d, J=3.7 Hz)
7.14 (1H, dd, J=9.9, 1.5 Hz)
7.25 (1H, d, J=3.7 Hz)
7.46 (1H, d, J=1.5 Hz)
7.80 (1H, d, J=9.9 Hz)

Industrial Applicability.

The present invention provides potent therapeutic or preventive drugs for pollakiuria and urinary incontinence, which do not have the side effects of anti-cholinergic drugs, anuresis and hydrodipsia, have excellent peroral absorbability with less inconsistency in the drug metabolism among individual patients because of the high bioavailability and are expected to have high effective rate. The present invention especially provides therapeutic or preventive drugs for pollakiuria and urinary incontinence with less side effects and superior stability in the body, which contain 4-isopropyl-2-methoxy-7-[4-(2-pyrimidyl)piperazinomethyl]-2,4,6-cycloheptatrien-1-one, 2-hydroxy-4-isopropyl-7-[4-(2-pyrimidyl)piperazinomethyl]-2,4,6-cycloheptatrien-1-one or pharmacologically acceptable salts thereof as the active ingredient compounds.

Also, these compounds induce drowsiness in mammals by oral administration but did not show affinity to the benzodiazepine receptor and the GABA receptor and did not demonstrate any muscle relaxing activity. Therefore the present invention provides short-term sleep inducers which do not cause side effects of benzodiazepine drugs.

Further, the present invention provides salts not so hygroscopic that it is easy to guarantee the contents of the active ingredient compounds in the formulation for production and supplying: the examples of such salts are 4-isopropyl-2-methoxy-7-[4-(2-pyrimidyl)piperazinomethyl]-2,4,6-cycloheptatrien-1-one 2 DL-tartrate or 4-isopropyl-2-methoxy-7-[4-(2-pyrimidyl)piperazinomethyl]-2,4,6-cycloheptatrien-1-one 1 fumarate.

## Claims

1. General formula (I)

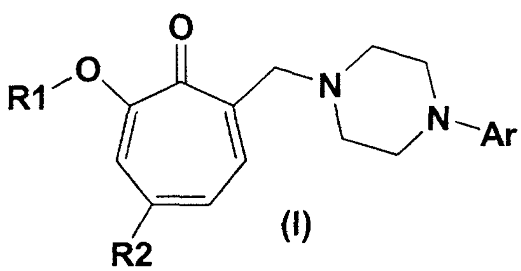

(I)

An oral therapeutic or preventive drug for pollakiuria and urinary incontinence which contains as an active ingredient compound represented in the general formula (I) [wherein R1 represents a hydrogen atom or an alkyl group with 1-3 carbon atoms in a linear or branched carbon chain, R2 represents a hydrogen atom or an isopropyl group and Ar represents an un-substituted heteroaromatic group with 2 hetero atoms] or pharmacologically acceptable salts thereof.

2. An oral therapeutic or preventive drug for pollakiuria and urinary incontinence according to claim 1, wherein Ar represents an un-substituted 2-pyrimidyl group or an un-substituted 2-thiazolyl group.

3. An oral therapeutic or preventive drug for pollakiuria and urinary incontinence according to claim 1, wherein the compounds represented in general formula (I) are 4-isopropyl-2-methoxy-7-[4-(2-pyrimidyl)piperazinomethyl]-

2,4,6-cycloheptatrien-1-one or 2-hydroxy-4-isopropyl-7-[4-(2-pyrimidyl)piperazinomethyl]-2,4,6-cycloheptatrien-1-one.

**4.** An oral therapeutic or preventive drug for pollakiuria and urinary incontinence according to claim 1, wherein the pharmacologically acceptable salt is DL-tartrate or fumarate.

**5.** An oral sleep inducer which contains the compounds according to any one of claims 1 - 4 or pharmacologically acceptable salts thereof as the active ingredient compounds.

**6.** A serotonin 1 A antagonist which contains the compounds according to any one of claims 1 - 4 or pharmacologically acceptable salts thereof as the active ingredient compounds.

**7.** 4-Isopropyl-2-methoxy-7-[4-(2-pyrimidyl)piperazinomethyl]-2,4,6-cycloheptatrien-1-one or 2-hydroxy-4-isopropyl-7-[4-(2-pyrimidyl)piperazinomethyl]-2,4,6-cycloheptatrien-1-one or 2-hydroxy-4-isopropyl-7-[4-(2-thiazolyl)piperazinomethyl]-2,4,6-cycloheptatrien-1-one or pharmacologically acceptable salts thereof.

**8.** 4-Isopropyl-2-methoxy-7-[4-(2-pyrimidyl)piperazinomethyl]-2,4,6-cycloheptatrien-1-one 2 DL-tartrate or 4-isopropyl-2-methoxy-7-[4-(2-pyrimidyl)piperazinomethyl]-2,4,6-cycloheptatrien-1-one 1 fumarate.

## INTERNATIONAL SEARCH REPORT

International application No.
PCT/JP03/06003

A. CLASSIFICATION OF SUBJECT MATTER
Int.Cl[7] A61K31/496, 31/506, A61P13/00, 13/02, C07D239/42, 277/42

According to International Patent Classification (IPC) or to both national classification and IPC

B. FIELDS SEARCHED

Minimum documentation searched (classification system followed by classification symbols)
Int.Cl[7] A61K31/496, 31/506, C07D239/42, 277/42

Documentation searched other than minimum documentation to the extent that such documents are included in the fields searched

Electronic data base consulted during the international search (name of data base and, where practicable, search terms used)
CA(STN), REGISTRY(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 99/00366 A (Nippon Kayaku Co., Ltd.), 07 January, 1999 (07.01.99), Full text & EP 995741 A | 1-4,6<br>5,7,8 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 June, 2003 (10.06.03) | 24 June, 2003 (24.06.03) |
| Name and mailing address of the ISA/ Japanese Patent Office | Authorized officer |
| Facsimile No. | Telephone No. |

Form PCT/ISA/210 (second sheet) (July 1998)